# EUROPÄISCHE PATENTSCHRIFT

(11) **EP 0 723 451 B1**
(45) Veröffentlichungstag und Bekanntmachung des Hinweises auf die Patenterteilung: **25.08.1999**
(21) Anmeldenummer: 94930168.3
(22) Anmeldetag: 13.10.1994
(51) Int. Cl.: A61K 33/14, A61K 33/00, A61K 38/55

(54) **VERWENDUNG VON LITHIUMVERBINDUNGEN IN KOMBINATION MIT ACE-HEMMERN UND/ODER ANGIOTENSIN II-REZEPTOR-ANTAGONISTEN ZUR BEHANDLUNG VON ERHÖHTEM BLUTDRUCK**
USE OF LITHIUM COMPOUNDS IN COMBINATION WITH ACE-INHIBITORS AND/OR ANGIOTENSIN II -RECEPTOR ANTAGONIST FOR TREATING HIGH BLOOD PRESSURE
UTILISATION DE COMPOSES DE LITHIUM EN COMBINAISON AVEC DES INHIBITEURS DE L'ACE ET/OU DES ANTAGONISTES DU RECEPTEUR A ANGIOTENSIN-II POUR TRAITER L'HYPERTENSION

(30) Priorität: 13.10.1993 DE 4334927; 13.10.1993 DE 4334936; 23.11.1993 DE 4339848
(43) Veröffentlichungstag der Anmeldung: 31.07.1996
(73) Patentinhaber: Lehmann, Karla, 70184 Stuttgart (DE)
(72) Erfinder: Lehmann, Karla, 70184 Stuttgart (DE)
(74) Vertreter: Weller, Wolfgang, Dr.rer.nat.
(86) Internationale Anmeldenummer: EP9403380
(87) Internationale Veröffentlichungsnummer: WO9510289

(56) Entgegenhaltungen:
- DE-A- 2 410 181
- GB-A- 2 113 064
- GB-A- 2 159 160
- US-A- 4 849 414
- US-A- 4 977 145
- PHYSIOL.BEHAV., Bd.44, Nr.1, 1988 Seiten 69 - 74 E.F.O'CONNOR 'Lithium Chloride Stabilizes Systolic Blood Pressure and Increases Adrenal Catecholamines in the Spontaneously Hypertensive Rat'
- BIULL.EKSP.BIOL.MED. (U.S.S.R.), Bd.91, Nr.2, Februar 1981 Seiten 139 - 142 E.V.LUKOSHKOVA ET AL. 'EFFECT OF LITHIUM DRUGS ON HEART ARRHYTHMIAS INDUCED BY LIGATION OF THE COMMON CAROTID ARTERIES'
- RES.COMMUN.CHEM.PATHOL.PHARMACOL., Bd.35, Nr.1, Januar 1982 Seiten 169 - 172 G.BAETGE ET AL. 'Chronic Lithium Decreases Blood Pressure in Spontaneously Hypertensive Rats'
- ARCH. ARZNEITHERAP., Bd.3, Nr.2, 1979 Seiten 127 - 136 E.SZIRMAI 'Monotherapie mit einer Chinin-Lithium-Salicylat-Kombination bei rheumatischen Erkrankungen und Beeinflussung einer gleichzeitig bestehenden primären essentiellen Hypertonie sowie arteriosklerotischen cerebralen Durchblutungsstörungen ohne Hypertonie als Zweiterkrankung'
- KLIN.WOCHENSCHR., Bd.45, Nr.13, 1. Juli 1967 Seiten 659 - 660 A.HEIZMANN ET AL. 'Der Einfluss von Lithiumchlorid auf die Blutdruckwirkung von Renin, Angiotensin und Noradrenalin bei Ratten'
- VERH.DTSCH.GES.INN.MED., Bd.73, 1967 Seiten 774 - 777 A.HEIZMANN ET AL. 'Der Einfluss von Lithiumchlorid auf die Blutdruckwirkung von renin, Angiotensin und Noradrenalin bei Ratten'
- AM.J.MED., Bd.85, Nr.6, Dezember 1988 Seiten 893 - 894 G.SIMON 'COMBINATION ANGIOTENSIN CONVERTING ENZYME INHIBITOR/LITHIUM THERAPY CONTRAINDICATED IN RENAL DISEASE'
- J.CLIN.PSYCHIATRY, Bd.53, Nr.11, November 1992 Seiten 398 - 400 K.DASGUPTA ET AL. 'The Effect of Enalapril on Serum Lithium Levels in Healthy Men'
- POSTGRAD.MED., Bd.87, Nr.1, Januar 1990 Seiten 223 - 243 P.AMADIO ET AL. 'ACE Inhibitors'
- Pschyrembel Klinisches Wörterbuch,1994,p,72
- Pharmazeutische Stoffliste,10 Auflage,p.185,69-70,84,25,56,94,131, 175,123,177-178,191,18-19,339,52,237-238.

## Beschreibung

Die Erfindung betrifft die Verwendung von Lithiumverbindungen in fixer Kombination mit einem ACE-Hemmer und/oder einem Angiotensin II-Rezeptor-Antagonisten zur Herstellung eines Arzneimittels zur Behandlung von erhöhtem Blutdruck und zur Verhütung von dadurch bedingten Folgeerkrankungen.

Bluthochdruck ist eine Volkskrankheit, die bekanntermaßen zusammen mit ihren Folgekrankheiten an der Spitze der Todesursachen steht. Bei 80 bis 95 % aller Hochdruckkrankheiten kann keine definitive Ursache gefunden werden; man nimmt eine multifaktorielle Genese an.

Bekannt ist die Abhängigkeit der Blutdruckhöhe von der sympathischen (adrenergenen) Aktivität, der Aktivität des Renin-Angiotensin-Aldosteron-Systemes (RAAS) und der Höhe der Serum-Natriumkonzentrationen (siehe Ganten, D. und Ritz E. (ed) "Lehrbuch der Hypertonie", Schattauer Verlag, Stuttgart-New York, 1985).

Jeder chronisch erhöhte Blutdruck muß behandelt werden. Eine medikamentöse Monotherapie erzielt in der Regel jedoch nur bei ca. 50 % der behandlungsbedürftigen Hochdruckkrankheiten eine ausreichende Blutdrucksenkung. Auch die in vergangener Zeit zur Behandlung von Bluthochdruck herangezogenen ACE-Hemmer (Angiotensin Converting Enzyme-Hemmer) zeigen in Monotherapie kein besseres Ansprechen, siehe dazu H. Breithaupt "Behandlung der Hypertonie", Schattauer Verlag, Stuttgart-New York, 1986, Seite 84.

Lithiumverbindungen werden bislang bei der Behandlung der Manie und zur Prophylaxe der manisch-depressiven Erkrankung eingesetzt, was auf Untersuchungen des Australiers Cade aus dem Jahre 1949 zurückgeht, siehe dazu C.-J. Estler "Lehrbuch der allgemeinen und systematischen Pharmakologie und Toxikologie", Schattauer Verlag Stuttgart - New York, 1986, Seite 166.

William E. Fann et al. "Effects of lithium on adrenergic function in man", Clin. Pharmacol. Ther. 13/1: 71-77 (1972) beschreiben eine Untersuchung zur Klärung des antimanischen Wirkungsmechanismus von Lithium. Für Lithium wurden antiadrenerge Wirkungen als Teil seiner antipsychotischen Wirksamkeit belegt.

A. Heizmann und D. Klaus haben in ihrer Untersuchung "Der Einfluß von Lithiumchlorid auf die Blutdruckwirkung von Renin, Angiotensin und Noradrenalin bei Ratten" Klin. Wschr. 45/13:659-660 (1967) an Ratten die Beeinflussung des Tonus der glatten Muskelzelle durch Veränderung der Ionenverteilung nach Lithiumgabe durchgeführt, und zwar zur Klärung der Frage, ob die Blutdruckwirkung von Angiotensin, Renin und Noradrenalin durch Änderung der Natrium-Verteilung beeinflußbar ist. Die Lithiumverabreichung bei diesen Untersuchungen an Ratten diente dazu, die Natriumkonzentration gezielt zu beeinflussen, um die daraus resultierende Veränderung des Natriumhaushaltes in Zusammenhang mit den Wirkungen von Angiotensin, Renin und Noradrenalin zu untersuchen.

K. Fleischer et al. "Beeinflussung des Plasmarenins und der pressorischen Ansprechbarkeit auf Angiotensinamid durch Lithium beim Menschen" Arzneim.-Forsch. 21/9:1363-1464 (1971) haben eine experimentelle Untersuchung an gesunden Probanden durchgeführt, um die von A. Heizmann bei Ratten erzielten Ergebnisse zu reproduzieren. Bei der Untersuchung von Fleischer wird eine Abschwächung der pressorischen Ansprechbarkeit von Angiotensin bei Verabreichung von Lithiumsalzen an gesunden Menschen festgestellt. Die Verabreichung von Lithium führt zu einer Störung des Natriumhaushaltes bzw. der Natriumbilanz, die dann zu einer Abschwächung der pressorischen Ansprechbarkeit führt.

Stefan R. Nahorski et al. "Lithium and the phosphoinositide cycle: an example of uncompetitive inhibition and its pharmacological consequences", Trends in Pharmacol.Sci. 12/8:297-303 (1991) geben eine Übersicht über die biochemischen Wirkungen von Lithiun auf den Inositolstoffwechsel zur Erklärung des Wirkungsmechanismus von Lithium bei manisch-depressiven Erkrankungen.

K.J. Catt et al. "The role of angiotensin II receptors in vascular regulation" J. Cardiovasc.Pharmacol. 6 Suppl.4; Seiten 575-586 (1984) geben eine allgemeine Übersicht über die Bedeutung von A II Rezeptoren für die Regulation der Gefäßweite. Es handelt sich dabei um Untersuchungen an Ratten, wobei Hinweise auf eine Reduktion der Bindung von A II durch Lithium an der A. mesenterica der Ratte gegeben wurden.

Douste-Blazy et al. "Angiotensin converting enzyme inhibitors and lithium treatment", Lancet I: Seite 1448 (1986) beschreiben einen Fall von Lithiumtoxizität bei Serumspiegeln in Höhe von 3,3 mmol/l nach Komedikation mit einem stark wirkenden ACE-Hemmer. Als Symptome der Vergiftung werden genannt: Ataxie, Dysarthrie, Tremor, Verwirrung, EEG-Veränderungen, Bradycardie, eingeschränkte Nierenfunktion.

G. J. Navis et al. "Volume homeostasis, angiotensin converting enzym inhibition, and lithium therapy" Am. J. Med. 86: 621 (1989) beschreiben einen Einzelfall einer Lithiumintoxikation bei einer 65jährigen Probandin nach 6-monatiger Komedikation mit Enalapril. Als Symptome der Intoxikation bei Lithiumspiegeln von 2,53 mmol/l werden genannt: Erhöhung der Stuhlhäufigkeit, Dehydratation, ein Blutdruck von 70/50 mmHg, Oligurie.

R.N. Santella et al. "Focal segmental glomerulosclerosis in patients receiving lithium carbonate" Am. J. Med. 84: 951-954 (1988) berichten über tödliche Ausgänge von Lithiumtoxikation nach Komedikationen mit Captopril. Die Symptomatik war: nephrotisches Syndrom, Niereninsuffizienz, Thrombose, Lungenembolie, Tod.

G. Simon "Combination angiotensin converting enzyme inhibitor/lithium therapy contraindicated in renal disease" Am. J. Med. 85: 893-894 (1988) berichtet ebenfalls, unter Bezug auf die Publikation von Santella über Fälle von Lithiumtoxizität nach Komedikation mit Enalapril. Die Symptomatik war Niereninsuffizienz, Proteinurie.

In der US-A-4 977 145 wird die kombinierte Verwendung eines ACE-Hemmers mit einem Antidepressivum wie z.B. Lithium zur Behandlung von Depressionen, also einer psychatrischen Erkrankung, beschrieben.

Aus der GB-A-2 159 160 ist bekannt, ein Lithiumsalz eines ACE-Hemmers im Laufe der Produktion von ACE-Hemmern herzustellen, um den ACE-Hemmer in Form des Salzes aus dem Reaktionsgemisch entfernen zu können.

Die US-A-4 849 414 offenbart Salze von ACE-Hemmern.

In J.Clin. Psychiatry, 53 (11), November 1992, Seiten 398 bis 400, wird eine Untersuchung vorgestellt, die die Auswirkung des ACE-Hemmers Enalapril auf Lithiumwerte im Serum von Patienten betrifft, denen Lithiumverbindungen als Antidepressiva und, weil diese möglicherweise auch an Bluthochdruck leiden, zugleich ein ACE-Hemmer verabreicht wurde. Diese Untersuchungen wurden an jungen gesunden Männern durchgeführt.

Aus der DE-A-2 410 181 ist bekannt, Lithiumorotat zur Behandlung von Gefäß- und Gewebserkrankungen, insbesondere im cerebralen Bereich einzusetzen.

Die GB-A-2 113 064 offenbart ein mineralisiertes Trinkwasser, das Strontium-, Magnesium-, Calcium- und Lithiumionen enthält.

In Physiol.Behav., 44 (1), 1988, Seiten 69 bis 74, wird eine Untersuchung beschrieben, die sich mit der Blutdruckstabilisierung durch Lithiumchlorid bei spontan hypertensiven Ratten beschäftigt. Nach subakuter Verabreichung wurde keine Blutdrucksenkung festgestellt.

Auch in Res.Commun.Chem.Pathol.Pharmacol., 35 (1), Januar 1982, 169 bis 172, wird die Auswirkung einer chronischen Lithiumbehandlung auf den Blutdruck von spontan hypertensiven Ratten beschrieben.

In Arch.Arzneitherap., 3 (2), 1979, Seiten 127 bis 136, wird eine Untersuchung vorgestellt, die sich mit der Wirkung des weit verbreiteten Arzneimittels "Togal®" beschäftigt. Dort wird angegeben, daß Togal möglicherweise auch als Antihypertensivum eingesetzt werden könnte.

Aufgabe der vorliegenden Erfindung ist es, neue Möglichkeiten zur Behandlung eines erhöhten Blutdrucks und zur Verhütung von dadurch bedingten Folgeerkrankungen zu schaffen.

Erfindungsgemäß wird die Aufgabe dadurch gelöst, daß die Lithiumverbindungen in fixer Kombination mit zumindest einem ACE-Hemmer, mit Ausnahme von Li-Salzen von ACE-Hemmern, und/oder in fixer Kombination mit zumindest einem Angiotensin II-Rezeptor-Antagonisten verwendet werden.

Es wurde festgestellt, daß durch die Verabreichung von Lithiumverbindungen in fixer Kombination mit einem ACE-Hemmer und/oder einem Angiotensin II-Rezeptor-Antagonisten ein erhöhter Blutdruck behandelt werden kann.

Dies ist insoweit als überraschend anzusehen, da Lithiumsalze eine verbreitete Anwendung als Psychopharmaka gefunden haben, eine synergistische blutdrucksenkende Wirkung von Lithiumverbindungen zusammen mit ACE-Hemmern und/oder einem Angiotensin II-Rezeptor-Antagonisten im Sinne eines Antihypertensivums aber noch nicht beobachtet wurde.

Die Addition eines zweiten Wirkstoffes mit blutdrucksenkender Wirkung verbessert den Effekt und erhöht die Ansprechquote der Behandlung.

ACE-Hemmer allein, zeigen, wie zuvor erwähnt (Breithaupt) in Monotherapie lediglich ein etwa 50%-iges Ansprechen. Der Wirkungsmechanismus der ACE-Hemmer beruht auf der Hemmung der enzymatischen Umwandlung von Angiotensin I in das pressorisch (Blutdruck steigernd) wirkende Angiotensin II. Dadurch sinkt auch die Aktivität von Aldosteron und die damit in Verbindung stehende Natrium-Reabsorption in der Niere, was zu einem Natriumverlust führt. Der Salzhunger wird bei sinkenden zentralen Angiotensin II-Wirkungen eingeschränkt. Die sich daraus summierende Hyponatriämie begünstigt die blutdrucksenkenden Eigenschaften der ACE-Hemmer in der Peripherie. Die Beziehung zwischen ansteigender Dosis und blutdrucksenkender Wirkung ist bei ACE-Hemmern schwach ausgeprägt, die optimale Wirkung stellt sich langsam innerhalb von ca. 3 bis 4 Wochen ein. Lithiumverbindungen wirken wesentlich rascher blutdrucksenkend, so daß in der fixen Kombination von Lithiumverbindungen und ACE-Hemmern eine sehr rasche blutdrucksenkende Wirkung erzielt werden kann.

Durch die erfindungsgemäße fixe Kombination eines ACE-Hemmers mit einer Lithiumverbindung werden alle bekannten Ursachen des erhöhten Blutdruckes bekämpft, bei gleichzeitiger verbesserter und verbreiterter Wirksamkeit der ACE-Hemmer. Bei Kombination beider Wirkstoffe, nämlich Lithiumverbindung und ACE-Hemmer kann die übliche Dosierung gesenkt werden, wodurch die Verträglichkeit des ACE-Hemmers verbessert wird, die bislang ein großes Problem bei der ACE-Hemmer-Therapie dargestellt hat.

Als besonders vorteilhaft hat sich herausgestellt, folgende Lithiumverbindungen einzusetzen, nämlich Lithiumazetat, -adipinat, -aspartat, -hydrogenaspartat, -benzoat, -bromid, -chlorid, -glukonat, -karbonat, -orotat, -salizylat, -sulfat, -zitrat.

Die notwendigen Dosen an Lithiumverbindungen liegen im unteren Bereich derer, wie sie zur Behandlung von Psychosen benötigt werden. Unerwünschte Reaktionen sind bislang nicht bekannt geworden. Somit erweist sich die Verwendung niedrig dosierter Lithiumverbindungen als Basis für ein physiologisches Antihypertensivum.

In zweckmäßiger Weise übersteigt die Dosierung der Lithiumverbindungen die bekannten üblichen Tagesdosen nicht. Die Verabreichung erfolgt bevorzugt oral oder intravenös.

Wegen der klinisch bedeutsamen Abhängigkeit des Blutdrucks vom Natriumhaushalt (hohes Serum-Natrium erhöht den Blutdruck) und der damit in engem Zusammenhang stehenden Aktivität des für den Blutdruck äußerst wichtigen ReninAngiotensin-Aldosteron-Systems RAAS (niedriges Serum-Natrium und/oder Volumenmangel aktiviert dieses System, hohes Serum-Natrium hemmt dieses System) sind alle pathophysiologischen Veränderungen und/oder die Gabe von Arzneimitteln mit Auswirkung auf den Natriumhaushalt und/oder die Aktivität des RAAS deshalb von besonderer Bedeutung. Die blutdrucksenkenden Wirkungen von Lithium beruhen insbesondere auf der Senkung adrenerger (sympathischer) Funktionen, auf der Senkung von Angiotensinwirkungen und auf der Beeinflussung des Natriumhaushaltes.

Unterstützt werden diese Effekte durch eine lithiumbedingte Hemmung der vasopressinabhängigen Wasserreabsorption in distalen Tubulus der Niere, woraus eine zusätzliche Kreislaufentlastung resultiert.

Als besonders vorteilhaft hat sich die Verwendung von Alacepril, Benazepril, Captopril, Cilazapril, Delapril, Enalapril, Fosinopril, Lisinopril, Moveltripril, Pentopril, Perindopril, Ramipril, Quinapril, Spirapril, Zofenopril als ACE-Hemmer herausgestellt.

Auch in der fixen Kombination von Lithiumverbindungen mit Angiotensin II-Rezeptor-Antagonisten konnte eine verbesserte und verbreiterte Wirksamkeit festgestellt werden.

Jüngere Untersuchungen von P.C. Wong "Nonpeptid angiotensin II receptor antagonists" Hypertension 15: 823 - 834 (1990) haben gezeigt, daß auch Angiotensin II-Rezeptor-Antagonisten in Monotherapie kein günstigeres Ansprechen als ACE-Hemmer zeigen. Ihr Wirkungsmechanismus beruht auf der Blockierung der pressorischen (blutdrucksteigernden) Wirkung von Angiotensin II. Die Beziehung zwischen ansteigender Dosis und blutdrucksenkender Wirkung ist bei Angiotensin II-Rezeptor-Antagonisten schwach ausgeprägt. Ihre optimale Wirkung stellt sich nach akuter Gabe langsam innerhalb von 4 bis 6 Stunden ein (siehe Y. Christen et al. "Oral administration of DuP 753, a specific angiotensin II receptor antagonist, to normal male volunteers", Circulation 83, 1333 - 1342, 1991). Durch die erfindungsgemäße fixe Kombination eines Angiotensin II-Rezeptor-Antagonisten mit einer Lithiumverbindung können alle bekannte Ursachen des erhöhten Blutdrucks bekämpft werden, bei gleichzeitiger verbesserter und verbreiterter Wirksamkeit der Angiotensin II-Rezeptor-Antagonisten. Durch die fixe Kombination kann die übliche Dosierung der Einzelkomponenten gesenkt werden, wodurch die Verträglichkeit steigt.

Dabei ist es bevorzugt, als Angiotensin II-Rezeptor-Antagonist Losartan oder wirksame Metaboliten der Angiotensin II-Rezeptor-Antagonisten zu verwenden.

Vorteilhafterweise werden die Wirkstoffe zusammen mit üblichen pharmazeutischen Trägern, Verdünnungsmitteln und/oder Hilfsstoffen verwendet.

Das molare Verhältnis der Lithiumverbindung zu den ACE-Hemmern und/oder Angiotensin II-Rezeptor-Antagonisten kann zwischen 100 bis 0,01 betragen, wobei das Verhältnis so abzustimmen ist, daß daraus eine Erhöhung der blutdrucksenkenden Wirksamkeit der Einzelkomponenten resultiert. Dabei wird auf den molaren Lithiumgehalt des entsprechenden Salzes bezogen.

Die Erfindung wird nachfolgend anhand einer Pilotstudie näher beschrieben und erläutert. In einer Pilotuntersuchung an vier Probanden (zwei Frauen und zwei Männer im Alter von 23 bis 52 Jahren), wurde der blutdrucksenkende Effekt zweier kumulierender Dosen Lithium (8 mmol und 16 mmol) an aufeinanderfolgenden Tagen überprüft. Die Lithiumverbindung wurde als Lithiumkarbonat verabreicht. Ferner wurde in die Untersuchung eine Patentin in hohem Lebensalter (87 Jahre) mit einseitiger Nephrektomie und erhöhten Blutdruckwerten einbezogen. Die Dosierung von Lithium wurde in diesem Fall auf 4 mmol beschränkt und am zweiten Versuchstag nicht erhöht. Alle Blutdruckmessungen erfolgten im Sitzen nach 5-minütiger Ruhepause mit einem Blutdruckmeßgerät "Supertronik" der Firma Medema am gleichen Arm. In die weiteren Berechnungen gingen der Mittelwert aus jeweils drei gemessenen Blutdruckwerten (systolisch und diastolisch) ein. Der Tagesablauf wurde bei allen Versuchspersonen in gleicher Weise eingehalten (Frühstück 9.30, Mittagessen 13.00, Abendessen 19.00 Uhr).

Während des Studienablaufs wurden körperliche Aktivitätsspitzen vermieden. Die Aufnahme von Genußmitteln mit Ausnahme der im Prüfplan vorgesehenen Genußmittel war untersagt. Zur Anhebung des Blutdruckes wurde am Vortag und an den beiden Versuchstagen jeweils um 15.30 Uhr starker schwarzer Tee verabreicht.

Die Auswertung erfolgte deskriptiv mittels der Funktionen "Mittelwert" und "Standardabweichung" (SD) von Harvard Graphics für Windows.

Am ersten Tag wurde zwischen 15.00 und 18.00 sowie zwischen 21.00 und 22.00 Uhr der Blutdruck stündlich gemessen.

Am zweiten Versuchstag wurde nach der morgendlichen Blutdruckmessung 9.00 Uhr die erste Lithiumdosis in Höhe von 8 mmol mit einer Tasse Kräutertee verabreicht. Zwischen 9.00 und 13.00 sowie zwischen 15.00 und 18.00 Uhr wurde der Blutdruck stündlich gemessen.

Tabelle 1 zeigt den Blutdruckverlauf von 4 Probanden am ersten Tag, also ohne die Verabreichung von Lithiumsalzen.

Tabelle 2 zeigt den Blutdruckverlauf derselben Probanden nach Verabreichung von 8 mmol Lithium.

Nach der ersten Dosis an Lithium sank der systolische Blutdruck im Mittel um 4,8 mmHg, der diastolische Blutdruck um 5,21 mmHg gegenüber dem gleichen Zeitraum des Vortages. Die Differenz zum Vortag (ohne Lithium) wurde deutlicher, wenn die Blutdruckwerte 16.00 und 17.00 Uhr (nach Provokation eines Blutdruckanstieges durch Tee) jeweils miteinander verglichen werden. Der systolische Blutdruckabfall betrug nach Verabreichung von Lithium 9,3 (16.00 Uhr-Wert), 9,67 (17.00 Uhr-Wert) mmHg, der diastolische Blutdruckabfall 6,97 (17.00 Uhr-Wert)-11,7 (16.00 Uhr-Wert) mmHg.

Wie aus der beiliegenden Tabelle 3 zu entnehmen, löst die zweite Dosis Lithium in Höhe von 16 mmol bereits am Morgen gegenüber den Werten des Vortages (8 mmol Lithium) eine deutliche Blutdrucksenkung aus (zwischen 7 - 12 mmHg systolisch und diastolisch bis zu 7,6 mmHg). Im Vergleich zum Vorwert (ohne Lithium) sank, gleiche Zeiträume betrachtet, der systolische Blutdruck im Mittel um 9,4 und der diastolische Blutdruck um 5 mmHg. Die 16.00 und 17.00 Uhr-Werte lagen systolisch mit 10,64 - 12,87 mmHg unter denen der Vorwerte, diastolisch um 6 - 9 mmHg.

Bei der Patientin (87 Jahre) ergab sich, wie das aus Tabelle 4 zu entnehmen ist, nach der ersten Dosis von Lithium (4 mmol) außer einer leichten diastolischen Blutdrucksenkung im Mittel kein blutdrucksenkender Effekt. Der durch Tee provozierte Blutdruckanstieg auf 145,33/107,33 mmHg wurde jedoch auf 133,3/80 mmHg reduziert. Die zweite Dosis Lithium in Höhe von ebenfalls 4 mmol (am nächsten Tag verabreicht) senkte im Mittel den systolischen Druck um 10,6 und den diastolischen Druck um 9,3 mmHg gegenüber den Blutdruckwerten ohne Lithium.

Bei der gleichzeitigen Verabreichung von Lithiumsalzen mit ACE-Hemmern und/oder Angiotensin II-Rezeptor-Antagonisten können geringere als bislang verwendete Dosen der Kombinationspartner eingesetzt werden, um in fixer Kombination eine synergistische Gesamtwirkung zu erzielen. Diese geht über die Einzelwirkung der Komponenten hinaus, so daß eine insgesamt wesentlich bessere Verträglichkeit unter Beibehaltung der raschen blutdrucksenkenden Wirkung des Lithiums erzielt wird. Durch den Synergiepartner Lithium kann jedwede Ursache des erhöhten Blutdrucks mit beeinflußt werden, also zusätzlich Wirkungen, die außerhalb des Wirkungsmechanismus von ACE-Hemmern und Angiotensin II-Rezeptor-Antagonisten liegen.

**Tabelle 1**

| **Blutdruckwerte ohne Verabreichung von Lithium (n = 4)** | | | | |
|---|---|---|---|---|
| Zeit | Mittelwert systolisch | SD | Mittelwert diastolisch | SD |
| 9.00 | 0 | 0 | 0 | 0 |
| 10.00 | 0 | 0 | 0 | 0 |
| 11.00 | 0 | 0 | 0 | 0 |
| 12.00 | 0 | 0 | 0 | 0 |
| 13.00 | 0 | 0 | 0 | 0 |
| 15.00 | 117,5425 | 6,387912 | 68,3175 | 9,007576 |
| 16.00 | 122,5025 | 11,83698 | 76,4925 | 3,039181 |
| 17.00 | 122,8425 | 10,46876 | 74,66 | 10,78153 |
| 18.00 | 121,725 | 11,12798 | 77,525 | 17,29109 |
| 21.00 | 114,1925 | 8,038823 | 70,9925 | 7,059013 |
| 22.00 | 120,3325 | 8,436034 | 76,5175 | 4,510972 |
| Tagessumme | 119,8563 ±10,05988 | | 74,08417 ±10,34794 | |

**Tabelle 2**

| **Blutdruckwerte nach Verabreichung von 8 mmol Lithium (n = 4)** | | | | |
|---|---|---|---|---|
| Zeit | Mittelwert systolisch | SD | Mittelwert diastolisch | SD |
| 9.00 | 105,65 | 8,65 | 72 | 2,44949 |
| 10.00 | 116,15 | 5,972227 | 69,1675 | 4,253136 |
| 11.00 | 111,9925 | 8,017772 | 69,1675 | 10,0717 |
| 12.00 | 109,46 | 5,538533 | 64,16 | 11,18711 |
| 13.00 | 119,085 | 6,373164 | 70,575 | 10,97004 |
| 15.00 | 114,8175 | 9,303167 | 66,65 | 12,00094 |
| 16.00 | 113,1925 | 9,932153 | 64,7925 | 9,580014 |
| 17.00 | 113,1675 | 8,203912 | 67,6925 | 13,53718 |
| 18.00 | 113,6 | 5,808184 | 71,4925 | 6,058186 |
| 21.00 | 114,9175 | 4,983515 | 71,5 | 15,17578 |
| 22.00 | 120,6675 | 9,055201 | 70,3175 | 7,346184 |
| Tagessumme | 113,8818 ±8,588026 | | 68,865 ±10,37893 | |
| vergl. Zeiten | 115,06 | | 68,74 | |

**Tabelle 3**

| **Blutdruckwerte nach Verabreichung von 16 mmol Lithium (n = 4)** | | | | |
|---|---|---|---|---|
| Zeit | Mittelwert systolisch | SD | Mittelwert diastolisch | SD |
| 9.00 | 109,65 | 10,20404 | 73,3175 | 11,63793 |
| 10.00 | 115,7425 | 17,05076 | 74,6575 | 11,1316 |
| 11.00 | 104,625 | 13,25413 | 61,5425 | 5,145427 |
| 12.00 | 100,39 | 10,60633 | 64,22333 | 10,07272 |
| 13.00 | 106,5425 | 7,530632 | 65,98 | 7,939578 |
| 15.00 | 104,6675 | 7,257869 | 63,335 | 1,054621 |
| 16.00 | 109,625 | 6,776568 | 67,5 | 7,150175 |
| 17.00 | 112,2 | 6,102868 | 68,5425 | 6,464551 |
| 18.00 | 109,16 | 4,286181 | 66,8275 | 8,408592 |
| 21.00 | 114,86 | 10,29016 | 72,7 | 15,26254 |
| 22.00 | 112,2 | 4,887399 | 75,33667 | 8,37987 |
| Tagessumme | 109,06 ±7,97649 | | 68,54 ±10,3353 | |
| vergl. Zeiten | 110,45 | | 69,04 | |

**Tabelle 4**

| **Blutdruckwerte einer 87-jährigen Patientin** | | | | | | |
|---|---|---|---|---|---|---|
| Zeit | VW syst. | VW diast. | 1x4 mmol syst. | 1x4 mmol diast. | 2.4 mmol syst. | 2.4 mmol diast. |
| 9.00 | 0 | 0 | 147,5 | 104,5 | 149,33 | 90 |
| 10.00 | 0 | 0 | 134 | 114,66 | 117 | 68,5 |
| 11.00 | 0 | 0 | 134 | 81,33 | 134 | 76,66 |
| 12.00 | 0 | 0 | 122,66 | 92 | 125,33 | 73,33 |
| 13.00 | 0 | 0 | 138 | 82 | 137,33 | 82,66 |
| 15.00 | 137,33 | 85,33 | 136,66 | 93,33 | 113,5 | 71,5 |
| 16.00 | 145,33 | 107,33 | 133,33 | 80 | 110 | 81,33 |
| 17.00 | 138,66 | 88 | 139,33 | 84 | 121,33 | 71,33 |
| 18.00 | 132,66 | 78 | 130 | 92 | 114,5 | 76 |
| 21.00 | 127,33 | 80,66 | 140,66 | 80,66 | 146,5 | 81 |
| 22.00 | 127,33 | 83,33 | 138 | 78 | 139,5 | 85,5 |
| Tagessumme | 134,77 ±8,27 | 87,11 ±9,91 | 136,17 ±8,62 | 89,76 ±11,62 | 127,78 ±14,22 | 77,78 ±7,68 |
| vergl. Zeiten | 134,77 | 87,11 | 136,3 | 84,6 | 124,22 | 77,78 |

## Patentansprüche

1. Verwendung von Lithiumverbindungen zur Herstellung eines Arzneimittels zur Behandlung von erhöhtem Blutdruck und zur Verhütung von dadurch bedingten Folgeerkrankungen, dadurch gekennzeichnet, daß die Lithiumverbindung in fixer Kombination mit zumindest einem ACE-Hemmer, mit Ausnahme von Li-Salzen von ACE-Hemmern, und/oder in fixer Kombination mit zumindest einem Angiotensin II-Rezeptor-Antagonisten verwendet wird.

2. Verwendung nach Anspruch 1, dadurch gekennzeichnet, daß Lithiumverbindungen, ausgewählt aus der Gruppe bestehend aus Lithiumazetat, -adipinat, -aspartat, -hydrogenaspartat, -benzoat, -bromid, -chlorid, -glukonat, -karbonat, -orotat, -salizylat, -sulfat, -zitrat verwendet werden.

3. Verwendung nach Anspruch 1 oder 2, dadurch gekennzeichnet, daß der zumindest eine ACE-Hemmer ausgewählt ist aus der Gruppe bestehend aus Alacepril, Benazepril, Captopril, Cilazapril, Delapril, Enalapril, Fosinopril, Lisinopril, Moveltipril, Pentopril, Perindopril, Ramipril, Quinapril, Spirapril, Zofenopril.

4. Verwendung nach einem der Ansprüche 1 bis 3, dadurch gekennzeichnet, daß als Angiotensin II-Rezeptor-Antagonist Losartan oder wirksame Metaboliten der Angiotensin II-Rezeptor-Antagonisten verwendet werden.

5. Verwendung nach einem der Ansprüche 1 bis 4, dadurch gekennzeichnet, daß die Wirkstoffe zusammen mit üblichen pharmazeutischen Trägern, Verdünnungsmitteln und/oder Hilfsstoffen verwendet werden.

6. Verwendung nach einem der Ansprüche 1 bis 5, dadurch gekennzeichnet, daß das molare Verhältnis zwischen Lithiumverbindung und ACE-Hemmer bzw. Angiotensin II-Rezeptor-Antagonist zwischen 100 und 0,01 liegt.

## Claims

1. Use of lithium compounds for the preparation of a medicament for the treatment of raised blood pressure and for the prevention of secondary diseases caused thereby, characterized in that the lithium compound is used in a fixed combination with at least one ACE inhibitor, excluding Li salts of ACE inhibitors, and/or in a fixed combination with at least one angiotensin II receptor antagonist.

2. Use according to Claim 1, characterized in that the lithium compounds used are selected from the group comprising lithium acetate, adipate, aspartate, hydrogenaspartate, benzoate, bromide, chloride, gluconate, carbonate, orotate, salicylate, sulfate and citrate.

3. Use according to Claim 1 or 2, characterized in that the ACE inhibitor (inhibitors) is (are) selected from the group comprising alacepril, benazepril, captopril, cilazapril, delapril, enalapril, fosinopril, lisinopril, moveltipril, pentopril, perindopril, ramipril, quinapril, spirapril and zofenopril.

4. Use according to one of Claims 1 to 3, characterized in that the angiotensin II receptor antagonist used is losartan or active metabolites of angiotensin II receptor antagonists.

5. Use according to one of Claims 1 to 4, characterized in that the active substances are used together with conventional pharmaceutical excipients, diluents and/or auxiliary substances.

6. Use according to one of Claims 1 to 5, characterized in that the molar ratio of lithium compound to ACE inhibitor or angiotensin II receptor antagonist is between 100 and 0.01.

## Revendications

1. Utilisation de composés de lithium pour préparer un médicament destiné au traitement de l'hypertension et à la prévention des séquelles de cette dernière, caractérisée en ce que le composé de lithium est utilisé en combinaison fixe avec au moins un inhibiteur de l'ACE, à l'exception des sels de lithium d'inhibiteurs de l'ACE, et/ou en combinaison fixe avec au moins un antagoniste des récepteurs de l'angiotensine II.

2. Utilisation selon la revendication 1, caractérisée en ce que l'on utilise des composés de lithium choisis dans le groupe comprenant les acétate, adipate, aspartate, hydrogénoaspartate, benzoate, bromure, chlorure, gluconate, carbonate, orotate, salicylate, sulfate, citrate de lithium.

3. Utilisation selon la revendication 1 ou la revendication 2, caractérisé en ce que l'inhibiteur de l'ACE au nombre d'au moins un est choisi dans le groupe comprenant les alacépril, bénazépril, captopril, cilazapril, délapril, énalapril, fosinopril, lisinopril, moveltripril, pentopril, périndopril, ramipril, quinapril, spirapril, zofénopril.

4. Utilisation selon l'une quelconque des revendications 1 à 3, caractérisée en ce que l'on utilise comme antagoniste des récepteurs de l'angiotensine II le losartane ou des métabolites actifs des antagonistes des récepteurs de l'angiotensine II.

5. Utilisation selon l'une quelconque des revendications 1 à 4, caractérisée en ce que les principes actifs sont utilisés ensemble avec des véhicules, diluants et/ou adjuvants pharmaceutiques usuels.

6. Utilisation selon l'une quelconque des revendications 1 à 5, caractérisée en ce que le rapport molaire entre le composé de lithium et l'inhibiteur de l'ACE ou l'antagoniste des récepteurs de l'angiotensine II se situe entre 100 et 0,01.
